# EUROPEAN PATENT APPLICATION

(11) **EP 1 350 790 A1**
(43) Date of publication of application: **08.10.2003**
(21) Application number: 01270527.3
(22) Date of filing: 13.12.2001
(51) Int. Cl.: C07D 207/46, C07D 417/12, C07B 41/12, C07B 43/00

(54) **PROCESS FOR PREPARING ACTIVE ESTERS**

(30) Priority: 14.12.2000 JP 2000380145
(71) Applicant: ONO PHARMACEUTICAL CO., LTD., Osaka-shi, Osaka 541-8526 (JP)
(72) Inventor: OKUYAMA, Shigehiro, Fukui R.Inst.Ono Pharm.Co.Ltd., Sakai-gun, Fukui 913-0032 (JP); MASAOKA, Hideo, Fukui R.Inst.Ono Pharm.Co.Ltd., Sakai-gun, Fukui 913-0032 (JP)
(74) Representative: Henkel, Feiler, Hänzel
(86) International application number: JP0110936
(87) International publication number: WO02048103

(57) **Abstract**

The present invention relates to a method for the preparation of active ester, characterized by subjecting to a reaction a mixture of a carboxylic acid, which is a starting material, reagent which forms active ester and a base or a starting material carboxylic acid.

The present invention provides an active ester useful as an intermediate of pharmaceutical drugs etc. in high yield using an inexpensive reagent, in one-pot reaction, without causing isomerization, regardless of the structure of starting material carboxylic acid.

## Description

### Technical Field

The present invention relates to a method for the preparation of active ester. More specifically, the present invention relates to a method for the preparation of active ester of carboxylic acid, which is an intermediate in the induction from carboxylic acid to amide, ester or alcohol.

The method of the present invention makes it possible to prepare active ester efficiently, which is an intermediate of an important compound as a pharmaceutical drug, etc.

### Background

As methods for the preparation of active ester from a carboxylic acid compound, the followings are known;
(1) a method using a condensing reagent,
(2) a method using a halogenating reagent, and
(3) a method using a mixed acid anhydride.

The method (1), wherein a condensing reagent is used, is widely used, for example, it is carried out in an organic solvent (chloroform, methylene chloride, dimethylformamide, diethyl ether, etc.) or without a solvent, in the presence or absence of a tertiary amine (pyridine, triethylamine, dimethylaniline, dimethylaminopyridine, etc.) using a condensing reagent [1-(3-dimethylaminopropyl)-3-ethylcarbodiimide (EDC) or a hydrochloride thereof, 1,3-dicyclohexylcarbodiimide (DCC), etc.] by subjecting to a reaction a reagent which forms active ester [N-hydroxysuccinimide (HOSu), N-hydroxybenzotriazole (HOBt), etc.].

However, EDC is expensive, and so it is not a preferable reagent for industrial mass synthesis. On the other hand, DCC is comparatively less expensive, but it is stimulant and DCC has a disadvantage that it generates dicyclohexylurea as a by-product in the progress of the reaction and it is difficult to remove it.

The method (2), wherein a halogenating reagent is used, is carried out in an organic solvent (chloroform, methylene chloride, diethyl ether, tetrahydrofuran, etc.) or without a solvent, by subjecting to a reaction carboxylic acid with a halogenating reagent (oxalyl chloride, thionyl chloride, etc.), followed by subjecting to a reaction thus obtained acid halogenated compound with a reagent which forms active ester.

This method requires two steps; one is to subject to a reaction carboxylic acid with a halogenating reagent, and the other is to subject to a reaction thus obtained acid halide with active ester reagent. That is to say, first step gives acid halide by reaction a halogenating reagent and the second step gives the solution of acid halide to remove excess halogenating reagent, followed by subjecting to a reaction with a reagent which forms active ester.

In the industrial mass synthesis, generally, reduction of operations and steps is extremely important and increase of them affects the price of the final product greatly. Therefore, fewer operations and steps is desired for industrial mass synthesis

Moreover, amino acid whose N-terminus is protected by a protective group sensitive to acid (t-butoxycarbonyl etc.) or a peptide containing it, then in the first acid-halogenation reaction, the protective group is very liable to be cut off. And it has another disadvantageous problem, subgenerating N-carboxy anhydride. Therefore, it is not suitable for industrial mass synthesis, to say nothing of small-scale reaction.

The method (3), wherein a mixed acid anhydride is used, for example, is carried out in an organic solvent (chloroform, methylene chloride, diethyl ether, tetrahydrofuran, etc.) or without a solvent, in the presence of a tertiary amine (pyridine, triethylamine, dimethylaniline, dimethylaminopyridine, etc.) by subjecting to a reaction acid halide (pivaloyl chloride, tosyl chloride, mesyl chloride, chloroethyl formate, chloroisobutyl formate, etc.) and subjecting to a reaction thus given mixed acid anhydride and a reagent which forms active ester.

This method also requires two steps; one is to subject to a reaction a carboxylic acid and acid halide, and the other is to subject to a reaction thus obtained mixed acid anhydride with active ester reagent.

This method is not suitable for industrial mass synthesis, because the intermediate (i.e. mixed acid anhydride) has high reactivity and is unstable and easily decomposed, so it is difficult to judge whether it is quantitatively generated, and because the reaction is very liable to cause adverse reaction such as dismutation etc. due to overheating in the progress of reactions.

From these above, a better method for the preparation of active ester from carboxylic acid, in fewer steps and efficiently, regardless of the structure of carboxylic acid, without using a troublesome condensing reagent, has been desired.

On the other hand, when an optically active carboxylic acid by the existence of asymmetric carbon atom is used as a starting material, isomerization of the carboxylic acid matters. Therefore, a method for the preparation of active ester without isomerization has been also investigated.

When amino acid or peptide compounds are used as a starting material, usually N-terminus thereof is protected. A method has been also investigated to prepare the target active ester without generating by-product which is cut off the protected amino acid or peptide in the progress of the reaction.

For example, (4) a method for the preparation of succinimide ester of N-terminus protected amino acid or peptide, i.e. activated ester using N,N'-disuccinimidylsulfite of the following formula 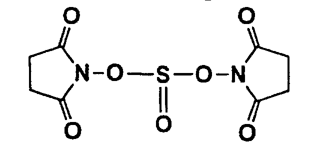 , which is prepared by subjecting to a reaction N-hydroxysuccinimide trimethylsilyl ether and thionyl chloride is proposed [Izv. Akad. Nauk SSSR, Ser. Khim. 5, 1163-5 (1984)].

And as another method for the preparation of N,N'-disuccinimidylsulfite, (5) N-hydroxysuccinimide, a method using thionyl chloride and triethylamine is also proposed [Izv. Akad. Nauk SSSR, Ser. Khim., 11, 2579-80 (1988)].

According to these methods, the active ester compound is obtained in low isomerization ratio and efficiently, but it requires several reaction steps.

Concretely, the method (4) requires 3 steps, (I) a process of preparing the starting material, N-hydroxysuccinimide trimethylsilyl ether, (ii) a process for the preparation of N,N'-disuccinimidylsulfite, using the ether, and (iii) a process for the preparation of target compound using carboxylic acid compound and N,N'-disuccinimidylsulfite.

On the other hand, the method (5) requires 2 steps, (I) a process of preparing N,N'-disuccinimidylsulfite from N-hydroxysuccinimide, and (ii) a process of preparing the target compound by the reaction of carboxylic acid compound and N,N'-disuccinimidylsulfite.

Both methods of (4) and (5) require purification after each reaction.

Such methods are not suitable for industrial mass synthesis, because they have a lot of steps.

Furthermore, in these methods one molecule of N-hydroxysuccinimide remains in the course of the reaction of carboxylic acid (N-terminus-protected amino acid or peptide) and N,N'-disuccinimidylsulfite, as shown in the following reaction scheme.

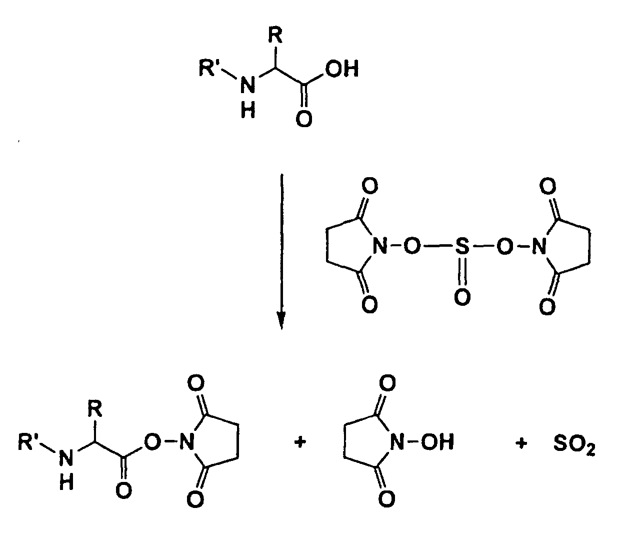

That is to say, it is not an advantageous method industrially because 1) it is inefficient because only half of N-hydroxysuccinimide in N,N'-disuccinimidylsulfite molecule is used in the reaction and 2) it is necessary to remove the by-product, i.e. N-hydroxysuccinimide after reaction.

Considering the above technical background, the following is desired; a method for the preparation of active ester from carboxylic acid, regardless of the structure of carboxylic acid, using an inexpensive reagent in fewer steps and in high yield. And when an optically active carboxylic acid is used, a method causing no isomerization is also desired.

### Disclosure of the present invention

The present invention relates to a method for the preparation of active ester of carboxylic acid, which is an intermediate from carboxylic acid to amide, ester or alcohol.

Concretely, the present invention relates to a method for the preparation of active ester in one-pot reaction using a reagent that forms active ester, a base and a halogenating reagent from carboxylic acid compound.

More concretely, the present invention relates to a method for the preparation of active ester, characterized by subjecting to a reaction a mixture of carboxylic acid (starting material), a reagent which forms active ester and a base in an organic solvent (for example, acetonitrile, tetrahydrofuran, dimethylformamide, dioxane, methylene chloride, ethyl acetate, ethyleneglycol dimethyl ether, 1,3-dimethyl-2-imidazolidinone, dimethysulfoxide, etc.) halogenating reagent, to prepare active ester in one-pot reaction.

As a reagent which forms active ester used in the present invention, N-hydroxysuccinimide (HOSu), N-hydroxybenzo triazole (HOBt), 3-hydroxy-4-oxo-3,4-dihydro-1,2,3-benzotriazine, N-hydroxy-5-norbornen-2,3-dicarboxylic acid imide, imidazole are used. Above all, N-hydroxysuccinimide is particularly preferable.

As a base, tertiary amine or aromatic heterocyclic amine is used, for example, triethylamine, diisopropyl ethylamine (DIPEA), dimethylaniline, N-methylmorpholine (NMM), dimethyaminopyridine (DMAP), pyridine, pyrimidine, pyrazine, pyridazine, quinoline, isoquinoline, quinazoline. Above all, triethylamine or pyridine is preferable particularly.

As a halogenating agent, for example, thionyl chloride, thionyl bromide, sulfuryl chloride, oxalyl chloride, phosgene, phosphorus oxychloride, diphosgene, triphosgene are used. Particularly, thionyl chloride or oxalyl chloride are preferably used.

The quantity of reagent which forms active ester, base and halogenating agent is as follows. Reagent which forms active ester is 1~1.5 eq., preferably 1~1.3 eq., base is 2~10 eq., preferably 2~5.5 eq., halogenating agent is 1~1.5 eq., preferably 1~1.3 eq., versus carboxylic acid (starting material) 1 eq.

The reaction is desirably carried out at a temperature of -20~40°C. When an optically active carboxylic acid is used as a starting material, -10~10°C is preferable and -5~5°C is particularly preferable.

The termination of the reaction is confirmed by thin layer chromatography, high performance liquid chromatography or other means for analysis.

Active ester compound, given after the reaction may be purified by normal purification techniques, for example, distillation under normal or reduced pressure, high performance liquid chromatography, thin layer chromatography, or column chromatography, washing or recrystallization.

According to the present invention, activated esters are prepared using an inexpensive halogenating reagent in a one-pot reaction from carboxylic acid without using a condensing agent which is expensive and has a lot of problems.

The method of the present invention is excellent, because it may be applied to various carboxylic acids regardless of the structures. For example, it may be applied to N-protected amino acid, its derivative and a peptide containing it without any difficulty, to say nothing of organic carboxylic acid. Particularly, when it is applied to even such amino acid, its derivative or a peptide containing it protected by protective group sensitive to acid, e.g. t-butoxycarbonyl etc., it has no danger to cause deprotection and generating by-products. And according to the present invention, the target active ester compound is prepared in high yield without causing any isomerization. According to the present invention, the target ester compound is prepared in high yield without causing isomerization.

And the active ester, which is prepared by the present invention, is isolatable and so it may be a synthetic intermediate for various compounds. For example, the active ester may be induced to amide, ester or alcohol, which is an important intermediate for pharmaceuticals etc and they are isolatable.

According to the present invention, reactions from a carboxylic acid to various amides, esters or alcohols may be carried out in a one-pot reaction, by subjecting to a reaction active ester without isolation with various compounds such as amine, alcohol or reductive reagent as it is.

### Industrial applicability

The method for the preparation of the present invention provides active ester in high yield, which is useful as an intermediate for pharmaceuticals etc. in one-pot reaction without causing isomerization, from organic carboxylic acid or amino acid whose N-terminus is protected by acid-sensitive protective group, regardless of the structure of starting material carboxylic acid.

### Best mode for carrying out the present invention

The present invention is illustrated by the examples, but is not limited to them.

The solvents described in the parentheses in NMR show the solvents used in the measurement. In the formula, Et is ethyl.

### Example 1

### (2R)-2-(t-butoxycarbonylamino)-3-cyclohexylmethylthio propanoic acid succinimide ester

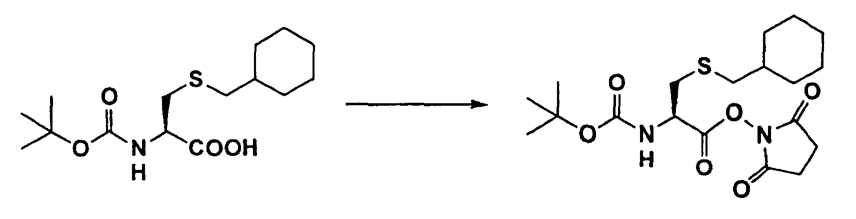

To a suspension of (2R)-2-(t-butoxycarbonylamino)-3-cyclohexylmethylthiopropanoic acid (952.8 mg; 100% e.e.) and N-hydroxysuccinimide (379.8 mg) in acetonitrile (1.28 mL) was added pyridine (1.22 ml), and the mixture was cooled to -10°C. To the mixture was added a solution of thionyl chloride (0.28 ml) in acetonitrile (0.4 ml) at 0°C dropwise, and the mixture was stirred for 30 minutes. The termination of the reaction was confirmed by HPLC. To the reaction solution was added cool water (11 ml) and was extracted with a mixture of t-butyl methyl ether (4 ml) and ethyl acetate (8 ml). The organic layer was washed with water (twice) and a saturated aqueous solution of sodium chloride, dried over anhydrous magnesium sulfate and was concentrated. The residue was dried overnight under reduced pressure to give the title compound (1.03 g; 82.8% yield, 86.2% purity, 100% e.e.). NMR (CDCl₃, 200 MHz): δ 5.32 (1H, bd), 4.82 (1H, bd), 3.16-2.94 (2H, m), 2.85 (2H, d, J = 6.8 Hz), 1.85-0.84 (30H, m) .
MS (FAB, Pos.):m/e 437(M⁺+Na), 415 (M⁺+ H).

### Example 2

### (4R)-3-t-butoxycarbonylthiazolidin-4-ylcarboxycic acid succinimide ester

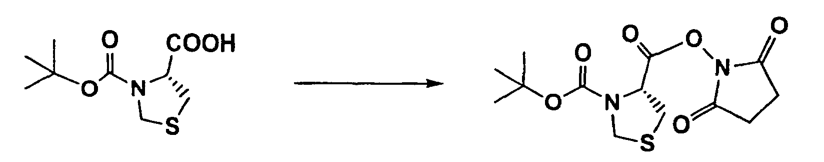

To a suspension of (4R)-3-butoxycarbonyl thiazolidin-4-ylcarboxylic acid (50 g; 100% e.e.) and N-hydroxy succinimide (25.9 g) in acetonitrile (146 ml) was added pyridine (38.1 ml) and the mixture was cooled to -4°C. To the mixture was added thionyl chloride (17.2 ml) in acetonitrile (20 ml) at a temperature of 0~2°C. The mixture was stirred for 1 hour under 0°C and the termination of the reaction was confirmed by HPLC. To the reaction solution was added cool water (630 ml) at 0°C dropwise and the mixture was stirred for 1 hours under 5°C. The precipitated crystal was collected by filtration and was washed with water twice. The crystal was washed with water until the filtrate became neutral. The obtained crystal was dried for 24 hours under reduced pressure to give the title compound (68.24 g; 96.4% yield, 99.2% purity, 100% e.e.) having the following physical data.
NMR (CDCl₃, 200 MHz): δ 4.93 (1H, m) , 4.61 (2H, dd, J = 28.7, 9.1 Hz), 3.60-3.38 (2H, m), 2.84 (4H, s), 1.48 (9H, s) .
MS (EI, Pos.): m/e 233 (M⁺), 189, 178, 160, 133, 105, 100.

### Example 3

### (4S)-4-benzyloxycarbonylamino-5-oxo-5-succinimidooxypentanoic acid ethyl ester

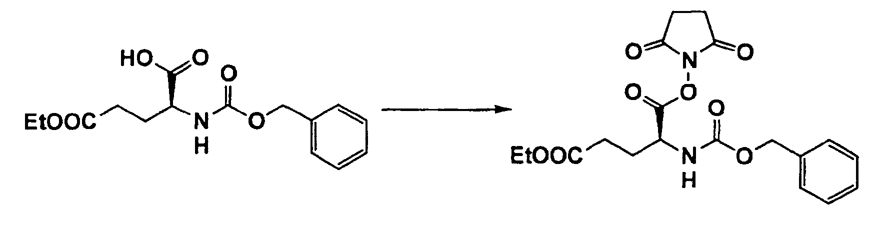

A suspension of (4S)-4-benzyloxycarbonylamino-4-carbonylpentanoic acid ethyl ester (50.0 g; 100% e.e.) and N-hydroxysuccinimide (20.46 g) in acetonitrile (100 ml) was cooled to 2°C and thereto was added pyridine (31.11 g) below 11°C. The mixture was cooled to -5°C and thereto was added a solution of thionyl chloride (24.72 g) in acetonitrile (27 ml) at a temperature below 0°C for 10 minutes. The termination of reaction was confirmed by HPLC. To the reaction solution was added cool water (620 ml) under 0°C and was extracted with a mixture of t-butyl methyl ether(210 ml) and ethyl acetate (410 ml). The organic layer was washed with water (twice) and a saturated aqueous solution of sodium chloride and was dried over magnesium sulfate and was concentrated. To the residue was added tetrahydrofuran and the mixture was concentrated. The operation was repeated. Part of the residue (2.016 g among 87.335 g) was dried overnight under reduced pressure to give the title compound (1.569 g, 98.6% yield, 98.2% purity, 100% e.e.) having the following physical data.
NMR (CDCl₃, 200 MHz) : δ 7.33 (5H, s), 5.63 (1H, bd, J = 9.5 Hz), 5.13 (2H, s), 4.87-4.72 (1H, m), 4.13 (2H, q, J = 7.5 Hz), 2.82 (4H, s), 2.53 (2H, t, J = 8.0 Hz), 2.50-2.07 (2H, m), 1.24 (3H, t, J = 7.5 Hz).

### Example 4

### 4-phenoxybenzoic acid succinimide ester

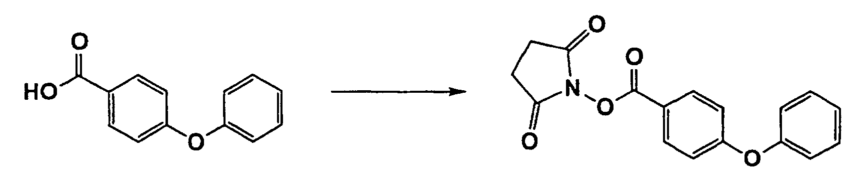

To a suspension of 4-phenoxybenzoic acid (533.6 mg) and N-hydroxysuccinimide (345.3 mg) in acetonitrile (1.9 ml) was added pyridine (0.6 ml) and the mixture was cooled in an ice bath. To the suspension was added thionyl chloride (0.22 ml) with internal temperature approximately 5°C and at the temperature the mixture was stirred for 10 minutes and the mixture was stirred for 50 minutes at room temperature. The termination of the reaction was confirmed by HPLC. To the reaction solution was added water (7.5 ml) and the mixture was cooled to 5°C and the mixture was stirred for 30 minutes. The precipitated crystals were collected and was washed with water 5 times and was dried under reduced pressure overnight to give the title compound (765 mg; 98.4% yield, 99.96% purity) having the following physical data.
NMR (CDCl₃, 200 MHz): δ 8.09 (2H, d, J = 10.5 Hz), 7.43 (2H, t, J = 8.5 Hz), 7.14 (1H, t, J = 8.5 Hz), 7.03 (4H, d, J = 10.5 Hz), 2.89 (4H, s).

### Reference example 1

### (2R)-N-(1-benzylpiperidin-4-yl)-2-(t-butoxycarbonylamino)-3-cyclohexylmethylthiopropanamide

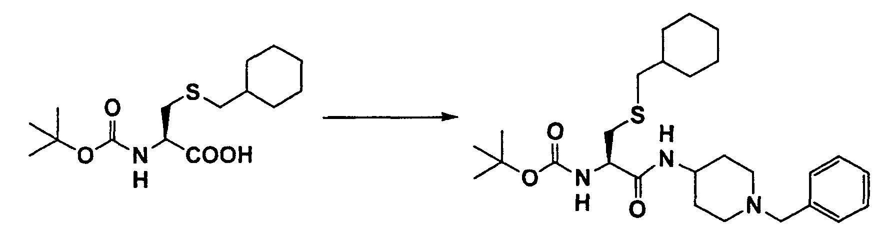

To a suspension of (2R)-2-(t-butoxycarbonylamino)-3-cyclohexylmethylthiopropanoic acid (952.4 mg; 100% e.e.) and N-hydroxysuccinimide (362.6 mg) in acetonitrile (1.28 ml) was added pyridine (1.22 ml) and the mixture was cooled to -10°C. To the mixture was added a solution of thionyl chloride (0.28 ml) in acetonitrile (0.4 ml) at 0°C dropwise, and the mixture was stirred for 30 minutes. The termination of the reaction was confirmed by HPLC. To the reaction solution was added a solution of 4-amino-1-benzylpiperidine (0.62 ml) and triethylamine (2.1 ml) in acetonitrile (3 ml) at a temperature below 3°C dropwise. The mixture was stirred for 30 minutes below 0°C and the termination of the reaction was confirmed by HPLC. To the reaction mixture was added water (30 ml) and was extracted with ethyl acetate. The organic layer was washed with a saturated aqueous solution of sodium bicarbonate and water, dried over magnesium sulfate and was concentrated. The residue was dried under reduced pressure for 4 hours to give the title compound (1.23 g; 86.9% yield, 82.7% purity, 97.2% e.e.) having the following physical data.
NMR (CDCl₃, 200 MHz) : δ 7.34-7.22 (5H, m), 4.16-4.11 (1H, m), 3.73-3.56 (1H, m), 3.51 (2H, s), 2.88-2.78 (3H, m), 2.67 (1H, dd, J = 13.8, 7.5 Hz), 2.43 (2H, d, J = 6.9 Hz), 2.17-2.09 (2H, m), 1.88-1.78 (4H, br), 1.76-1.38 (15H, m), 1.33-1.09 (3H, m), 1.00-0.87 (2H, m).
MS (MALDI, Pos.): m/e 490 (M + H⁺).

### Reference Example 2

By subjecting to amidation reaction an amine compound given by subjecting to the same method of reference example 3 or reference example 7 and active ester given by example 2, using the compound prepared in reference example 1, (2R)-N-(1-benzylpiperidin-4-yl)-3-cyclohexylmethylthio-2-((4R)-3-t-butoxycarbonylthiazolidin-4-ylcarbonylamino) propanamide of the following formula

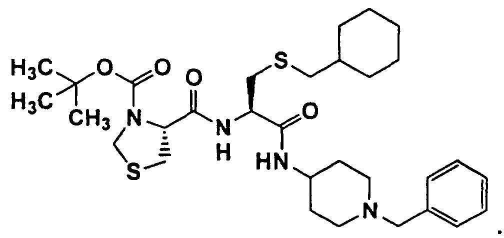

The compound is important as a pharmaceutical drug, described as a compound having N-type calcium channel inhibitory activity in the specification of WO00/00470.

### Reference Example 3

By subjecting to amidation reaction the active ester prepared in example 3 and the amine compound prepared by the same method of example 39 (only the part of reaction with sodium borohydride) → example 41 → example 69 (the part of refluxing after addition of 10% palladium on carbon, filtration and concentration) described in the specification of WO99/19296, followed by example 70 → example 71 in the specification, N-hydroxy-(2S)-methyl-5-ethoxymethoxy-(4S)-[N-(4-phenoxyohenylcarbonyl)amino]pentanamide of the following formula 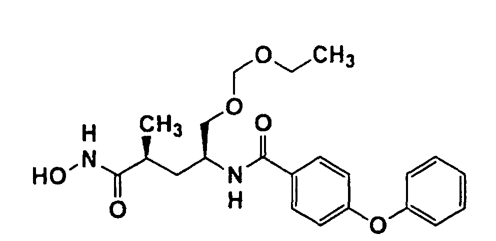 was given. The compound is important as a pharmaceutical drug, which is described as a compound having metalloproteinase inhibitory activity in the specification of WO99/19296.

### Reference Example 4

By subjecting to amidation reaction the amine prepared by the same method as example 39 (only the part of subjecting to a reaction with sodium borohydride) → example 69 (the part of refluxing after addition of 10% palladium on carbon, filtration and concentration) using the active ester prepared in example 3 and the active ester prepared in example 4 in the present invention, followed by the same method as example 45 → example 70 → example 48 → example 71 in the specification, N-hydroxy-(2S)-methyl-5-hydroxy-(4S)-[N-(4-phenoxyphenylcarbonyl)amino]pentanamide of the following formula 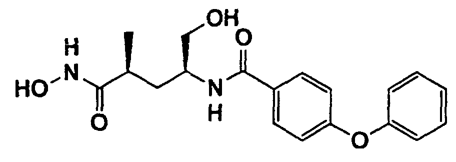 was given. The compound is important, which is described as a compound (pharmaceutical drug) having metalloproteinase inhibitory activity in the specification of WO99/19296.

## Claims

1. A method for the preparation of active ester, comprising adding a halogenating agent to a mixture of carboxylic acid, a reagent to form active ester and a base in an organic solvent and subjecting the mixture to a reaction in one-pot reaction.

2. The method for the preparation according to claim 1, which further comprises isolating the active ester after the reaction.

3. The method for the preparation of active ester according to claim 1, wherein the reagent which forms active ester is N-hydroxysuccinimide.

4. The method for the preparation of active ester according to claim 1, wherein the base is pyridine or triethylamine.

5. The method for the preparation of active ester according to claim 1, wherein the halogenating reagent is thionyl chloride or oxalyl chloride.

6. The method for the preparation of active ester according to claim 1, wherein 1~1.5 eq. of the reagent which forms active ester, 2~10 eq. of a base and 1~1.5 eq. of halogenating agent are used versus 1 eq. of starting material carboxylic acid.

7. The method for the preparation of active ester according to claim 1, wherein starting material is carboxylic acid is amino acid, its derivative or a peptide containing it, whose N-terminus is protected.

8. The method for the preparation of a reagent which forms active ester according to claim 1, wherein starting material carboxylic acid is (2R)-2-(t-butoxycarbonylamino)-3-cyclohexylmethylthiopropanoic acid.

9. The method for the preparation of a reagent which forms active ester according to claim 1, wherein starting material carboxylic acid is (4R)-3-t-butoxycarbonylthiazolizin-4-ylcarboxylic acid.

10. The method for the preparation of a reagent which forms active ester according to claim 1, wherein the starting material carboxylic acid is (4S)-4-benzyloxycarbonyl amino-4-carboxypentanoic acid ethyl ester.

11. The method for the preparation of active ester compound according to claim 1, wherein starting material carboxylic acid is 4-phenoxybenzoic acid.
